# EUROPÄISCHE PATENTSCHRIFT

(11) **EP 1 031 553 B1**
(45) Veröffentlichungstag und Bekanntmachung des Hinweises auf die Patenterteilung: **08.01.2003**
(21) Anmeldenummer: 00103368.7
(22) Anmeldetag: 22.02.2000
(51) Int. Cl.: C07C 67/343, C07C 51/353, C07C 69/593, C07C 55/14, C07B 37/00

(54) **Verfahren zur Herstellung von substituierten Olefinen**
Process for the preparation of substituted olefins
Procédé pour la préparation d'oléfines substituées

(30) Priorität: 22.02.1999 DE 19907519
(43) Veröffentlichungstag der Anmeldung: 30.08.2000
(73) Patentinhaber: BASF AKTIENGESELLSCHAFT, 67056 Ludwigshafen (DE)
(72) Erfinder: Schwab, Peter, Dr., 67098 Bad Dürkheim (DE); Schulz, Michael, Dr., 67067 Ludwigshafen (DE)
(74) Vertreter: Isenbruck, Günter, Dr.

(56) Entgegenhaltungen:
- WO-A-94/23836
- DE-A- 3 229 419
- E. VERKUILEN: "Metathesis of low-molecular unsaturated acid esters" RECUEIL DES TRAVAUX CHIMIQUES DES PAYS-BAS, Bd. 96, Nr. 11, 1977, Seiten 86-90, XP000914754 AMSTERDAM NL
- CHEMICAL ABSTRACTS, vol. 101, no. 2, 9. Juli 1984 (1984-07-09) Columbus, Ohio, US; abstract no. 7786y, "Adipic acid and its dimethyl ester" Seite 9; Spalte 2; XP002140575 & JP 59 029634 A (SUMITOMO CHEMICAL) 16. Februar 1984 (1984-02-16)

## Beschreibung

Die Erfindung betrifft ein Verfahren zur Herstellung von substituierten Olefinen durch Selbstmetathese oder Kreuzmetathese.

Bei der Olefinmetathese (Disproportionierung) findet in ihrer einfachsten Form eine reversible, metallkatalysierte Umalkylidenierung von Olefinen durch Bruch und Neuformierung von Kohlenstoff-Kohlenstoff-Doppelbindungen statt. Im Fall der Metathese acyclischer Olefine unterscheidet man beispielsweise zwischen einer Selbstmetathese, bei der ein Olefin in ein Gemisch zweier Olefine unterschiedlicher molarer Massen übergeht (beispielsweise Umsetzung von Propen zu Ethen und 2-Buten), und der Kreuz- oder Co-Metathese, die eine Umsetzung zweier unterschiedlicher Olefine beschreibt (beispielsweise von Propen mit 1-Buten zu Ethen und 2-Penten). Zu den weiteren Anwendungsgebieten der Olefinmetathese zählen Synthesen ungesättigter Polymere durch ringöffnende Metathesepolymerisation (ROMP) von cyclischen Olefinen und die acyclische Dien-Metathesepolymerisation (ADMET) von α,ω-Dienen. Neuere Anwendungen betreffen die selektive Ringöffnung von cyclischen Olefinen mit acyclischen Olefinen, sowie Ringschlußreaktionen (RCM), mit denen -vorzugsweise aus α, ω-Dienen - ungesättigte Ringe verschiedener Ringgröße hergestellt werden können.

Als Katalysatoren für Metathesereaktionen eignen sich prinzipiell homogene und heterogene Übergangsmetallverbindungen.

Heterogene Katalysatoren, beispielsweise Molybdän-, Wolfram- oder Rheniumoxide auf anorganischen oxidischen Trägern, zeichnen sich bei Reaktionen von nichtfunktionalisierten Olefinen durch hohe Aktivität und Regenerierbarkeit aus, müssen allerdings beim Einsatz funktionalisierter Olefine, wie Methyloleat, zur Aktivitätssteigerung oft mit einem Alkylierungsmittel vorbehandelt werden. Olefine mit protischen funktionellen Gruppen (wie Hydroxylgruppen, Carboxylgruppen oder Aminogruppen) führen zur spontanen Desaktivierung des Heterogenkontaktes.

Die vorliegende Erfindung betrifft ein Verfahren zu Herstellung von difunktionalisierten C₆-Kohlenwasserstoffen des Typs ECH₂CH=CHCH₂E, beispielsweise Adipinsäure und Derivaten davon, wobei zum Aufbau der C₆-Einheit eine Metathesereaktion eines Olefins vom Typ RCH=CHCH₂E als Schlüsselschritt durchgeführt wird.

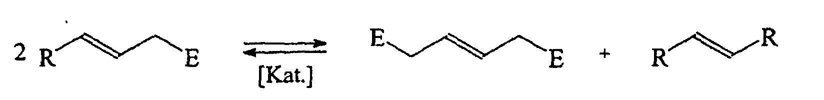

C₆-Kohlenwasserstoffes dieses Typs stellen nach Funktionalisierung industriell relevante Vor- und Zwischenprodukte dar: Adipinsäure beispielsweise dient als Vorprodukt für die Herstellung von Nylon 6.6 (Fasersektor) und wird bislang größtenteils durch oxidative Spaltung von Cyclohexan hergestellt. Neuere Entwicklungen betreffen Aufbaureaktionen für Adipinsäure aus Butadien, zum Beispiel nach Monsanto durch Carbonylierung der Zwischenstufe 1,4-Dimethoxy-2-buten und nach BASF durch zweistufige Carbonylierung von Butadien in Gegenwart von Methanol.

Die zweifache Carbonylierung erfordert drastische Reaktionsbedingungen und liefert, ausgehend von Butadien, mit ca. 70 % nur recht mäßige Ausbeuten an Adipinsäure über beide Stufen.

Daher erscheint die obige Metathesereaktion als Zugang zu den gewünschten Verbindungen als eine mögliche Alternative.

Die allgemein hohe Aktivität von homogenen Metathesekatalysatoren gegenüber Olefinen ist bei Einsatz elektronenarmer Olefine, wie Acrylsäure oder deren Derivaten, drastisch reduziert. Vor allem Selbstmetathesereaktionen von Olefinen des Typs RCH=CH(CH₂)ₙE zu RCH=CHR und E(CH₂)ₙCH=CH(CH₂)ₙE sind mit den bekannten Metathesekatalysatoren dann problematisch, wenn E ein elektronenziehender Substituent ist, n null oder 1 ist und R = H, Alkyl oder Aryl ist. Anwendungen substituierter Olefine wie 3-Pentensäuremethylester, 3-Pentensäure oder 3-Pentennitril in Selbstmetathesereaktionen sind demzufolge aufgrund zu geringer Aktivität kaum in der Literatur beschrieben.

J. Chem. Soc., Chem. Commun. 1983, 262-263, J. Cem. Soc., Chem. Commun. 1981, 1081-1082 und J. Organomet. Chem. 1985, 280, 115-122 beschreiben die Selbstmetathese ungesättigter Nitrile des Typs CH₂=CH(CH₂)ₙCN in Gegenwart von Re₂O₇/Al₂O₃-Heterogenkontakten, welche mit SnMe₄ oder SnEt₄ aktiviert wurden. Während 4-Pentennitril bis ca. 90 % umgesetzt wird, erfolgt mit Allylcyanid mit Ausnahme einer Isomerisierung zu Crotonnitril keine produktive Metathesereaktion.

Recl. Trav. Chim. Pays-Bas 1977, 96(11), 86-90 beschreibt Metathesereaktionen niedermolekularer ungesättigter Ester mit dem homogenen Katalysatorsystem WCl₆/SnMe₄. 3-Pentensäuremethylester wird zwar bei 2 mol-% WCl₆/SnMe₄ mit einer Selektivität von 95 % zu 2-Buten und Dehydroadipinsäureester umgesetzt. Nachteilig ist allerdings die hohe Empfindlichkeit des Katalysatorsystems gegenüber Feed-Verunreinigungen. Metathesereaktionen mit ungesättigten Säuren sind mit dem genannten Katalysatorsystem nicht möglich.

J. Mol. Catal. 1992, 76, 181-187 befaßt sich mit der Metathese funktionalisierter Olefine mit dem Katalysatorsystem WCl₆ (oder WOCl₄) 1,1,3,3-Tetramethyl-1,3-disilacyclobutan (DSBC). 4-Pentensäuremethylester wird im besten Versuch mit WOCl₄/DSBC bei Umsätzen von 54 % mit 94 % Selektivität zum entsprechenden C₈-Diester umgesetzt. In Gegenwart des gleichen Katalysatorsystems wird Allylcyanid unter Ethen-Extrusion bei 53 % Umsatz mit 82 % Selektivität zu Dehydroadipodinitril umgesetzt.

Chem. Lett. 1976, 1021-1024 beschreibt die Selbstmetathese von 4-Pentensäuremethylester bei Einsatz von WCl₆/Me₂Al₂Cl₂ mit 60 % Umsatz.

Es stellte sich die Aufgabe, einen wirtschaftlich attraktiven Syntheseweg für difunktionalisierte C₆-Kohlenwasserstoffe aus einfach zugänglichen Startmaterialien bei moderaten Reaktionsbedingungen unter Einsatz eines geeigneten, allgemein anwendbaren Katalysatorsystems zu entwickeln.

Die Aufgabe wird erfindungsgemäß gelöst durch ein Verfahren zur Herstellung von C₆-Verbindungen der allgemeinen Formel (I)

E-CH₂-CH=CH-CH₂-E¹ (I)

durch Selbstmetathese oder Kreuzmetathese von Verbindungen der allgemeinen Formeln (II) und/oder (III)

R-CH=CH-CH₂-E (II)

R¹-CH=CH-CH₂-E¹ (III)

mit den Bedeutungen
- E, E¹: unabhängig voneinander -CHO, -COOH, -COOR², -C(O)NR³R⁴, -CN
- R, R¹: unabhängig voneinander H, C₁₋₁₂-Alkyl, C₆₋₁₂-Aryl oder C₇₋₁₃ Alkylaryl
- R², R³, R⁴: unabhängig voneinander H, C₁₋₁₂-Alkyl, C₇₋₁₃-Aralkyl,
in Gegenwart eines Katalysators,
wobei ein homogener Katalysator, der Rutheniumverbindungen oder -komplexe enthält, eingesetzt wird.

Die Aufgabe wird damit erfindungsgemäß durch eine Verfahrenssequenz gelöst, in deren Schlüsselschritt zum Aufbau eines C₆-Kohlenwasserstoffs vom Typ ECH₂CH=CHCH₂E eine Selbstmetathesereaktion eines Olefins vom Typ RCH=CHCH₂E gemäß der nachfolgenden Gleichung durchgeführt wird.

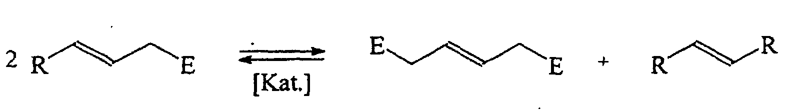

Als Nebenkomponente entsteht RCH=CHR in stöchiometrischen Mengen und kann gegebenenfalls durch Folgereaktionen weiterverarbeitet werden. Beispielsweise können durch Ethenolyse von RCH=CHR α-Olefine des Typs CH₂=CHR erhalten werden.

E stellt in der obigen Gleichung eine Aldehyd-, Ester-, Säure-, Säureamid- oder Nitrilfunktion dar. R entspricht Wasserstoff, Alkyl-, Aryl- oder Alkylarylresten. Als Alkylreste R bevorzugt sind lineare C₁₋₆-Alkylreste, wie Methyl oder Ethyl, oder verzweigte C₁₋₆-Alkylreste, bei denen die Verzweigung mindestens eine Methylengruppe von der Doppelbindung entfernt liegt.

Ferner ist es möglich, daß Substrate mit unterschiedlichen Resten R, R¹ und E, E¹ im Sinne einer Kreuzmetathesereaktion miteinander reagieren. In diesem Fall ist mit gemischten Reaktionsausträgen zu rechnen.

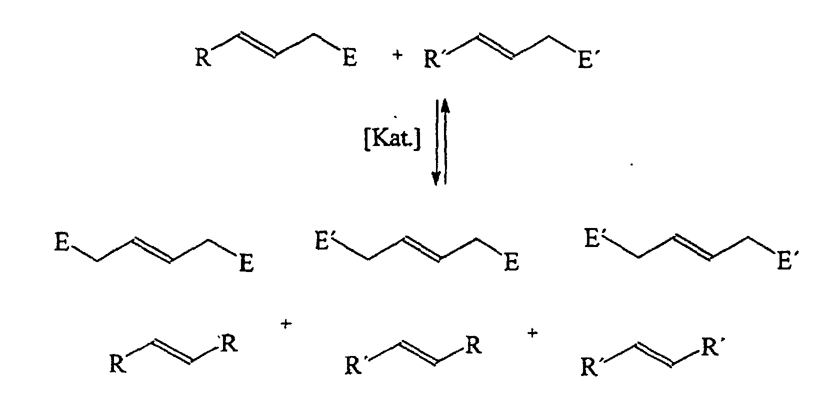

Vorzugsweise ist E = E' und R = R'. Besonders bevorzugt sind E und E' Esteroder Carbonsäurereste. R und R' sind vorzugsweise Methyl- oder Ethylgruppen.

Das erfindungsgemäße Verfahren wird in Gegenwart eines homogenen Katalysators, der Rutheniumverbindungen oder -komplexe enthält, durchgeführt. Dabei werden als Katalysator vorzugsweise Ruthenium-Alkylidenkomplexe eingesetzt. Vorzugsweise sind die Ruthenium-Alkylidenkomplexe ausgewählt aus 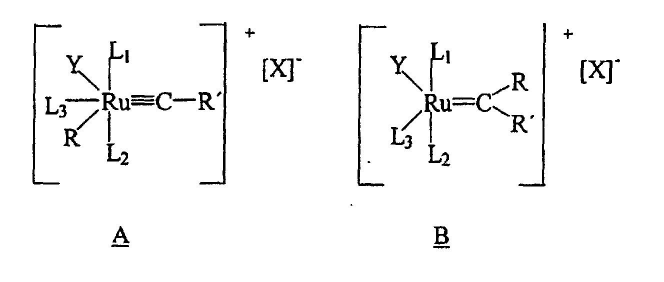 wobei B durch einen weiteren Liganden L₄ stabilisiert sein kann und
- X: ein nicht oder schwach an das Metallzentrum koordinierendes Anion,
- Y: einen ein- oder mehrzähnigen anionischen Liganden,
- R und R': jeweils unabhängig voneinander Wasserstoff oder einen, gegebenenfalls substituierten, C₁₋₂₀-Alkyl-, C₆₋₂₀-Aryl- oder C₇₋₂₀-Alkylarylrest und
- L₁, L₂, L₃ und L₄: unabhängig voneinander neutrale Elektronendonor-Liganden bedeuten,
oder
Rutheniumkomplexen der allgemeinen Formeln C oder D

RuX'Y'(=CH-CH₂R")L¹L² (C)

RuX'Y'(=CHR")L¹L² (D)

in der
- X',Y': die gleichen oder unterschiedliche anionische Liganden sind,
- R": Wasserstoff oder ein, gegebenenfalls substituierter, C₁₋₂₀-Alkylrest oder C₆₋₂₀-Arylrest ist, und
- L¹ und L²: unabhängig voneinander neutrale Elektronendonor-Liganden sind.

Vorzugsweise sind die neutralen Elektronendonor-Liganden Phosphane, Arsane, Stibane mit mindestens zwei sterisch anspruchsvollen Resten, Amine, Pyridine, π-koordinierte Olefine oder Lösemittelmoleküle. Besonders bevorzugt sind die neutralen Elektronendonor-Liganden ausgewählt aus Phosphanen der allgemeinen Formel PR^{a}R^{b}R^{c}, in der R^{a} und R^{b} unabhängig voneinander Phenylreste oder organische sterisch hindernde Reste sind und R^{c} Wasserstoff, ein, gegebenenfalls substituierter C₁₋₁₂-Alkyrest oder C₆₋₂₀-Arylrest oder wie für R^{a} definiert ist.

R^{a} und R^{b} sind vorzugsweise ausgewählt aus i-Propyl, tert-Butyl, Cyclopentyl, Cyclohexyl, Phenyl oder Menthyl.

Derartige Komplexe sind beispielsweise in WO 93/20111, WO 96/04289, WO 96/06185, WO 97/03096, wie auch in der DE-A-197 36 609 und DE-A-198 00 934 beschrieben.

Die kationischen Katalysatorsysteme enthalten als Aktivkomponenten kationische Rutheniumkomplexe der allgemeinen Formeln A (kationische Carbinkomplexe) oder B (kationische Carbenkomplexe) oder diese enthaltende Gemische 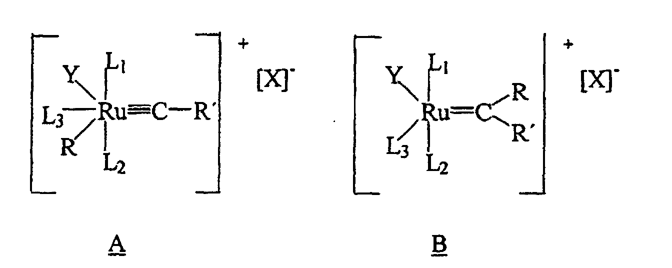 wobei B durch einen weiteren Liganden L₄ stabilisiert sein kann. In den Strukturen A und B bedeuten
- X⁻: ein nicht oder schwach an das Metallzentrum koordinierendes Anion, wie Komplexanionen aus der III. bis VII. Hauptgruppe des Periodensystems der Elemente, beispielsweise BR"₄⁻ (R"=F, Phenylreste, die durch ein oder mehrere Fluoratome oder perfluorierte C₁₋₆-Alkylreste substituiert sein können, wie C₆H₅₋ₙFₙ mit n = 1 bis 5), PF₆⁻, AsF₆⁻, SbF₆⁻, ClO₄⁻, CF₃SO₃⁻ oder FSO₃⁻,
- Y: einen ein- oder mehrzähnigen anionischen Liganden
- R und R': jeweils unabhängig voneinander Wasserstoff oder einen, gegebenenfalls substituierten, C₁₋₂₀-Alkyl-, C₆₋₂₀-Aryl- oder C₇₋₂₀-Alkylaryl oder Aralkylrest,

L₁, L₂, L₃ und L₄ unabhängig voneinander neutrale Elektonendonor-Liganden, vorzugsweise Stickstoff-Donoren, wie Amine und Pyridine, Phosphane, Arsane, Stibane mit mindestens zwei sterisch anspruchsvollen Resten, wie i-Propyl, t-Butyl, Cyclopentyl, Cyclohexyl, Menthyl oder ähnliches, aber auch Π-koordinierte Olefine oder Lösemittelmoleküle.

Vorzugsweise haben die Reste folgende Bedeutungen:
- X⁻: BR"₄⁻ mit R" = F oder C₆H₃ (m - CF₃)₂,
- Y: Halogen, vorzugsweise Chlor, oder OR mit R = C₁₋₆-Alkyl, C₆₋₁₂-Aryl, vorzugsweise Phenolat,
- R: H
- R': C₁₋₆-Alkyl, C₆₋₁₂-Aryl, C₇₋₂₀-Aralkyl, vorzugsweise Methyl oder Benzyl,
- L₁, L₂: Phosphane mit mindestens zwei sterisch anspruchsvollen Resten,
- L₃, L₄: cyclische oder acyclische Ether oder tertiäre Amine wie NMe₂Phenyl, NMe₃, NEt₃.

Die Synthese der Aktivkomponenten A und/oder B beziehungsweise von Gemischen, die diese Aktivkomponenten enthalten, kann ausgehend von zahlreichen metallorganischen Ausgangsstoffen erfolgen, beispielsweise
- durch Umsetzung von Hydrido(vinyliden)komplexen des Typs RuY(H)(=C=CHR)L₁L₂, deren Synthese durch Umsetzung von Ru-ClH(H₂)L₁L₂ mit terminalen Alkinen HC≡CR erfolgen kann, mit R⁺X⁻, wobei X⁻ ein nicht oder schwach koordinierendes Anion darstellt. RuClH(H₂)L₂ kann hierbei gemäß Literaturangaben beispielsweise aus dem polymeren Ruthenium-Precursor [RuCl₂(COD)]ₓ (COD = Cyclooctadien) in i-Propanol in Gegenwart von L unter Wasserstoffatmosphäre (Werner et al., Organometallics 1996, 15, 1960 bis 1962) oder ausgehend vom selben Startmaterial in sec-Butanol in Gegenwart von L und tert. Aminen (NEt₃) unter Wasserstoffatmosphäre (Grubbs et al., Organometallics 1997, 16, 3867 bis 3869) hergestellt werden. RuClH(H₂)L₂ ist ferner ausgehend von RuCl₃·H₂O in THF durch Umsetzung mit L in Gegenwart von aktiviertem Magnesium unter Wasserstoffatmosphäre (BASF AG, prioritätsältere, nicht vorveröffentlichte DE-A-198 00 934) zugänglich und wird vorzugsweise in situ mit 1-Alkinen zu den entsprechenden Hydrido(chloro)vinylidenkomplexen RuClH(=C=CHR)L₂ umgesetzt. Letztere können isoliert werden oder reagieren in situ mit H⁺X⁻ (X⁻ = nichtkoordinierendes Anion) zu den erfindungsgemäßen Aktivkomponenten A und/oder B.
- Durch Umsetzung von Verbindungen des Typs RuYY'(=CHR)L₁L₂ (wobei Y = Y' sein kann) mit R⁺X⁻, wobei X⁻ ein nicht- oder schwach koordiniertes Anion darstellt. Gemischt-anionische Alkylidenkomplexe RuXY(=CHCH₂R)L₂ können gemäß DE-A-198 00 934 ausgehend von RuXH(=C=CHR)L₂ hergestellt werden.
- Durch Umsetzung von Verbindungen des Typs RuYY'(=CHR)L₁L₂ mit anionenabstrahierenden Metallsalzen M⁺X⁻ beziehungsweise Lewissäuren, wie BF₃ oder AlCl₃, in Gegenwart eines Liganden L₃, wobei X⁻ ein nicht oder nur schwach koordinierendes Anion darstellt und die anionischen Liganden Y und Y' gleich oder voneinander verschieden sein können. MX kann beispielsweise AgPF₄, AgB(C₅F₅)₄, AgPF₆ oder AgSbF₆ sein.

R⁺X⁻, M⁺X⁻ und die entsprechenden Lewissäuren werden vorzugsweise, bezogen auf das metallorganische Edukt, im Molverhältnis 1:10 bis 1000:1 eingesetzt.

Umsetzungen zu den Aktivkomponenten A und/oder B werden vorzugsweise in organischen Lösungsmitteln unter Inertgasatmosphäre durchgeführt, vorzugsweise in solchen Lösemitteln, die ein ungesättigtes Metallzentrum durch Koordination stabilisieren können, wie aliphatische oder cyclische Ether wie Dioxan oder THF, Amine, DMSO, Nitrile, Phosphane, Arsane, Stibane, Wasser, Olefine oder sonstige Zweielektronendonoren. Bevorzugt wird die Reaktion in THF bei Temperaturen von -100 bis +100°C, bevorzugt -80 bis -40°C und Drücken von 1 mbar bis 100 bar, bevorzugt bei 0,5 bis 5 bar durchgeführt.

Die Umsetzung kann mit einem oder mehr Moläquivalenten R⁺X⁻ erfolgen. Bei Einsatz von überschüssigem R⁺X⁻ gebildetes L₁₋₃RX beinträchtigt die Reaktion nicht. Die hierbei erhaltenen, die Aktivkomponenten A und/oder B enthaltenden Zusammensetzungen können in situ als hochaktives Metathese-Katalysatorsystem eingesetzt werden oder bei tiefen Temperaturen unter Inertgasatmosphäre gelagert werden. Gegebenenfalls werden die Aktivkomponenten A oder B in isolierter Form eingesetzt.

In der Regel ist die Reaktion nach 1 s bis 10 h, vorzugsweise nach 3 s bis 1 h beendet. Als Reaktionsgefäße eignen sich allgemein Glas- oder Stahlbehälter, welche gegebenenfalls mit Keramik ausgekleidet werden.

Die Herstellung der Rutheniumkomplexe der allgemeinen Formel (C)

RuX'Y'(=CH-CH₂R")L¹L² (C)

in der
- X', Y': die gleichen oder unterschiedliche anionische Liganden sind,
- R": Wasserstoff oder ein, gegebenenfalls substituierter, C₁₋₂₀-Alkylrest oder C₆₋₂₀-Arylrest ist, und
- L¹ und L²: unabhängig voneinander neutrale Elektronendonor-Liganden sind,
erfolgt vorzugsweise durch
(a) Umsetzung von RuX₃ mit L¹ und L² in einem inerten Lösungsmittel in Gegenwart eines Reduktionsmittels und von Wasserstoff mit Verbindungen der allgemeinen Formel IV

   R"-C≡CH (IV)

   in der R" die oben angegebene Bedeutung hat, gegebenenfalls in Gegenwart von Wasser,
   zu einer Verbindung der allgemeinen Formel V

   RuX'H(=C=CHR")L¹L² (V)

   mit den vorstehenden Bedeutungen für X', R", L¹, L²,
(b) Abtrennung der Verbindung der allgemeinen Formel V vom Reaktionsgemisch und anschließende Umsetzung mit HY', (HL¹)Y' oder (HL²)Y' in einem inerten Lösungsmittel mit Verbindungen der allgemeinen Formel IV

   R"-C≡CH (IV)

   in der R" die oben angegebene Bedeutung hat, gegebenenfalls in Gegenwart von Wasser,
(c) anschließende Umsetzung mit HY', [HL¹]Y' oder [HL²]Y'.

Es wurde gefunden, daß die vorstehenden Rutheniumkomplexe direkt aus RuX'₃, vorzugsweise RuCl₃·3(H₂O) durch einfache Reaktion mit Liganden L¹ und L², Wasserstoff und terminalen Alkinen der allgemeinen Formel IV in Gegenwart von Reduktionsmitteln ohne Isolierung von Zwischenstufen in sehr guten Ausbeuten erhalten werden können. Die Rutheniumkomplexe enthalten dabei keine vinylischen Substituenten am Carbenkohlenstoffatom. Die Ausgangsstoffe sind kostengünstig herstellbar und leicht verfügbar.

Zur Herstellung der gemischt-anionischen Komplexe der allgemeinen Formel (C) wird die Zwischenstufe der allgemeinen Formel V gewonnen beziehungsweise isoliert und anschließend weiter umgesetzt. Dadurch können unterschiedliche Liganden X' und Y' eingebracht werden.

Zunächst erfolgt die Umsetzung von RuX'₃ mit den Liganden L¹ und L² in einem inerten Lösungsmittel in Gegenwart eines Reduktionsmittels und von Wasserstoff. Als Lösungsmittel können dabei Aromaten, Heteroaromaten, cyclische oder acyclische Ether eingesetzt werden. Bevorzugte Lösungsmittel sind Toluol, NMP, Tetrahydrofuran, Dialkylether, Glykolether und Dioxan. Besonders bevorzugt ist Tetrahydrofuran.

Als Reduktionsmittel kann jedes Reduktionsmittel eingesetzt werden, das unter den Reaktionsbedingungen Ru(III) zu Ru(II) reduziert Vorzugsweise wird die Reduktion mit Wasserstoff in Gegenwart eines metallischen oder nichtmetallischen Reduktionsmittels, bevorzugt in Gegenwart eines Alkalimetalls, Erdalkalimetalls oder Übergangsmetalls, wie Palladium oder Zink, das in metallischer Form vorliegt und/oder auf einen Träger aufgebracht sein kann, durchgeführt. Die Erdalkalimetalle, vorzugsweise Magnesium, werden vorzugsweise in einer aktivierten Form eingesetzt. Dabei kann die Aktivierung beispielsweise durch Kontaktieren mit einem chlorhaltigen organischen Lösungsmittel erhalten werden. Beispielsweise kann in einer Eintopfreaktion unter Inertgasatmosphäre Magnesium in einem verdünnten chlorhaltigen organischen Lösungsmittel, beispielsweise Dichlorethan, vorgelegt und nach einer Induktionsperiode von einer Sekunde bis 10 Stunden, vorzugsweise einer Minute bis einer Stunde mit dem Lösungsmittel, RuX'₃ und den Liganden L¹ und L² unter einer Wasserstoffatmosphäre umgesetzt werden. Dabei beträgt die Temperatur in diesem Umsetzungsschritt (a) vorzugsweise 0 bis 100°C, besonders bevorzugt 20 bis 80 °C, insbesondere 40 bis 60°C. Der Druck beträgt dabei vorzugsweise 0,1 bis 100 bar, besonders bevorzugt 0,5 bis 5 bar, insbesondere 0,8 bis 1,5 bar. Die Umsetztung erfolgt für einen Zeitraum von vorzugsweise 10 Minuten bis 100 Stunden, besonders bevorzugt eine Stunde bis 10 Stunden. Das Molverhältnis von Liganden L¹ und L² zusammengenommen zum eingesetzten Rutheniumsalz beträgt vorzugsweise 2 bis 20: 1, besonders bevorzugt 2 bis 5 : 1. Nach der Umsetzung im Schritt (a) wird das Reaktionsgemisch vorzugsweise bei einer Temperatur im Bereich von -80 bis 100°C, besonders bevorzugt -40 bis 50°C, insbesondere -30 bis 20°C mit einem 1-Alkin umgesetzt. Dabei beträgt das molare Verhältnis von ursprünglich eingesetztem Rutheniumsalz zu 1-Alkin vorzugsweise 1 : 1 bis 1 : 10. Die Umsetzung erfolgt vorzugsweise bei einem Druck von 0,1 bis 10 bar, besonders bevorzugt 0,8 bis 1,5 bar, insbesondere 1 bis 1,4 bar für einen Zeitraum von vorzugsweise 30 Sekunden bis 10 Stunden, besonders bevorzugt eine Minute bis eine Stunde.

In den Rutheniumkomplexen der allgemeinen Formeln (C) ist X' ein einzähniger anionischer Ligand, beispielsweise Halogen, Pseudohalogen, Carboxylat, Diketonat. Besonders bevorzugt bedeutet X' Halogen, insbesondere Brom oder Chlor, speziell Chlor. Besonders bevorzugt wird bei der Umsetzung RuCl₃·3H₂O eingesetzt.

In den Rutheniumkomplexen der allgemeinen Formel (C) kann Y' der gleiche Ligand wie X' sein. Vorzugsweise ist er ein von X' verschiedenes Halogen oder ein an ein Polymer oder einen Träger gebundenes Carboxyl, mit dem eine Träger-Fixierung des Katalysators möglich ist. Bei den Zwischenprodukten der allgemeinen Formel V kann auch ein Austausch des Liganden X' durch Salzmetathese mit MY' erfolgen, wobei M Alkalimetall oder Ammonium, vorzugsweise Kalium bedeutet. Auf diese Weise sind auch Produktgemische zugänglich.

L¹ und L² sind wie vorstehend beschriebene neutrale Elektronendonor-Liganden. Der Rest R ist Wasserstoff oder ein, gegebenenfalls substituierter, C₁₋₂₀-, vorzugsweise C₁₋₆-Alkylrest oder C₆₋₂₀-, vorzugsweise C₆₋₈-Arylrest. Besonders bevorzugt sind als Rutheniumkomplex der allgemeinen Formel (C) die Komplexe RuCl₂(=CH-CH₃)(PCy₃)₂ und RuCl₂(=CH-CH₂-Ph)(PCy₃)₂ wobei Cy einen Cyclohexylrest und Ph einen Phenylrest bedeuten.

Die Rutheniumkomplexe der allgemeinen Formel

RuX'₂(=CH-CH₂R")L¹L²

in der
- X': ein anionischer Ligand ist,
- R": Wasserstoff oder ein, gegebenenfalls substituierter C₁₋₂₀-Alkylrest oder C₆₋₂₀-Arylrest ist, und
- L¹ und L²: unabhängig voneinander neutrale Elektronendonor-Liganden sind,
können auch erhalten werden durch
a) Umsetzung von RuX'₃ mit einem Dien in einem Lösungsmittel auf Basis eines oder mehrerer aliphatischer sekundärer Alkohole in Gegenwart oder Abwesenheit eines Reduktionshilfsmittels, und sodann mit L¹ und L² in Gegenwart mindestens einer koordinierenden schwachen Base und von Wasserstoff und ohne Isolierung von Zwischenstufen
b) anschließende Umsetzung mit Verbindungen der allgemeinen Formel

   R"-C≡CH

   in der R" die oben angegebene Bedeutung hat, in Gegenwart einer löslichen Chloridquelle.

Gegenüber den im Stand der Technik beschriebenen Katalysatorsystemen zeichnen sich die erfindungsgemäß eingesetzten Rutheniumverbindungen u.a. dadurch aus, daß bereits bei sehr geringen Katalysatorkonzentrationen (100 ppm - 1 %) unter milden Reaktionsbedingungen (T = 0 bis 200°C, p = 1 bar absolut) hohe Selektivitäten bei vergleichsweise hohen Katalysator-Standzeiten erzielt werden können.

Bei Einsatz interner Olefine RCH=CHCH₂E mit R = Me oder Et kann die Zudosierung von Ethylen gemäß nachfolgender Gleichung zur Erhöhung des Umsatzes erforderlich oder zumindest hilfreich sein. In diesem Fall kann Ethylen als Strippgas eingesetzt werden.

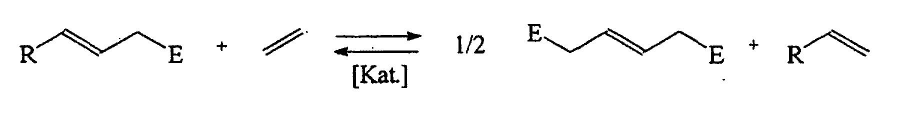

Der Zusatz von Lösungsmitteln, wie beispielsweise Pentan, Aceton, Ether und Toluol ist in keiner der beschriebenen Reaktion erforderlich, stört die Reaktion allerdings auch nicht.

Die Reaktionen werden bei Temperaturen von 0 bis 200°C sowie bei Drücken von 0.01 bis 100 bar durchgeführt und sind in der Regel nach 10 min bis 100 h beendet.

Die Umsetzungen können kontinuierlich und diskontinuierlich in Reaktoren, wie Glasgefäßen, Kesseln, Rohrreaktoren oder Umlaufreaktoren ausgeführt werden. Da es sich um Gleichgewichtsreaktionen handelt, ist es vorteilhaft, die Verfahrensprodukte im Sinne der Erzielung eines möglichst hohen Umsatzes möglichst rasch aus dem Gleichgewicht zu entfernen. Dies ist insbesondere für Reaktionen, bei denen als Nebenkomponenten Leichtsieder wie Ethen, 2-Buten oder Propen entstehen, geeignet.

Zur Isolierung der Verfahrensprodukte wird der Reaktionsaustrag, welcher gegebenenfalls im Verfahrensprodukt gelösten oder suspendierten Katalysator enthält, destillativ aufgearbeitet, wobei das Verfahrensprodukt nach Feindestillation isoliert werden kann. Der katalysatorhaltige Destillationssumpf kann in die Reaktion zurückgeführt werden. Ferner kann der Katalysator in einem hochsiedenden Lösungsmittel zurückgeführt werden. Denkbar ist auch, daß das erfindungsgemäße Verfahren in einer Reaktivdestillations-Vorrichtung ausgeübt wird, um in situ gebildete Leichtsiederkomponenten im Sinne der Maximierung des Umsatzes aus dem Gleichgewicht zu entfernen.

Die für die Metathesereaktion als Edukte eingesetzten Verbindungen des Typs RCH=CHCH₂E (R und E siehe oben) können u.a. aus einfach verfügbaren Startmaterialien wie Dienen, beispielsweise Butadien, durch Hydroformylierung, Carbonylierung, Hydrocyanierung in hohen Ausbeuten erhalten werden.

Die weitere Verarbeitung der im Metatheseaustrag befindlichen difunktionalisierten C₆-Kohlenwasserstoffe kann u.a. erfolgen durch Hydrierung, Hydroformylierung, reduktive Aminierung, Oxidation oder Ringschluß.

Die Erfindung wird durch die folgenden Beispiele näher erläutert.

### Beispiel 1:

### Synthese von Dehydroadipinsäuremethylester (C₆-Diester)

In einem Schlenkrohr werden 100 g (0,88 mol) 3-Pentensäuremethylester (3-PSE) bei unterschiedlichen Temperaturen und Atmosphärendruck mit 677 mg (0,9 mmol) RuCl₂(=CHMe)(PCy₃)₂ umgesetzt. Binnen weniger Minuten beobachtet man eine charakteristische Farbänderung der Lösung von violett nach weinrot. Der Reaktionsraum bleibt während der gesamten Versuchsdauer verschlossen, so daß gebildete leichtsiedende Nebenkomponenten nicht entweichen können. Nach unterschiedlichen Reaktionszeiten werden Proben entnommen und gaschromatographisch analysiert. Die Ergebnisse sind in den nachfolgenden Tabellen zusammengefaßt:
T = 20°C

| | 1 h | 5 h | 20h |
|---|---|---|---|
| Umsatz_{3-PSE} | 9 | 12 | 18 |
| Selektivität_{C6-Diester} | 100 | 100 | 99 |

T = 40°C

| | 1h | 5 h | 20h |
|---|---|---|---|
| Umsatz_{3-PSE} | 13 | 15 | 18 |
| Selektivität_{C6-Diester} | 99 | 99 | 98 |

T = 80°C

| | 1 h | 5 h | 20 h |
|---|---|---|---|
| Umsatz _{3-PSE} | 20 | 21 | 2 |
| Selektivität_{C6-Diester} | 99 | 98 | 97 |

### Beispiel 2:

### Synthese von Dehydroadipinsäure (C₆-Disäure)

In einem Schlenkrohr werden 100 g (1,0 mol) 3-Pentensäure bei 80°C und Atmosphärendruck mit 760 mg (1,0 mmol) RuCl₂(=CHMe)(PCy₃)₂ umgesetzt. Der Reaktionsraum bleibt während der gesamten Versuchsdauer verschlossen, so daß gebildete leichtsiedende Nebenkomponenten nicht entweichen können. Binnen weniger Minuten beobachtet man eine charakteristische Farbänderung der Lösung von violett nach weinrot. Nach 5 h wird der Reaktionsaustrag gaschromatographisch analysiert.
Umsatz_{3-Pentensäure} = 23 %
Selektivität_{C6-Disäure} = 98 %

### Beispiel 3:

### Synthese von Dehydroadipinsäuremethylester (C₆-Diester) in Gegenwart von Ethylen

In einem Gaseinleitungsschlenkrohr werden 100 g (0,88 mol) 3-Pentensäuremethylester bei Raumtemperatur mit 677 mg (0,9 mmol) RuCl₂(=CHMe)(PCy₃)₂ umgesetzt, und in die Lösung wird ein leichter Ethylenstrom eingeleitet. Binnen weniger Minuten beobachtet man eine charakteristische Farbänderung der Lösung von violett nach weinrot. Mit dem anhaltenden Ethylenstrom wird binnen 1 h gebildetes Propylen aus der Lösung ausgestrippt. Abschließend wird der Reaktionsaustrag gaschromatographisch analysiert.
Umsatz_{3-Pentensäure} = 45 %
Selektivität_{C6-Diester} = 98 %

### Beispiel 4:

### Synthese von Dehydroadipinsäuremethylester (C₆-Diester) bei 100 mbar

In einem Rundkolben mit aufgesetztem Tropftrichter werden 100 g (0,88 mol) 3-Pentensäuremethylester bei 40°C mit 677 mg (0,9 mmol) RuCl₂(=CHMe)(PCy₃)₂ versetzt und bei einem Unterdruck von 100 mbar zur Entfernung gebildeten 2-Butens über einen Zeitraum von 1 h allmählich mit weiteren 400 g (3,51 mol) 3-Pentensäuremethylester versetzt. Die Reaktionsmischung wird bei 40°C noch 1 h gerührt und abschließend destillativ aufgearbeitet.
Ausbeute_{C6-Diester} = 211 g (isoliert, 56 % der Theorie)

## Patentansprüche

1. Verfahren zur Herstellung von C₆-Verbindungen der allgemeinen Formel (I)
E-CH₂-CH=CH-CH₂-E¹ (I)
durch Selbstmetathese oder Kreuzmetathese von Verbindungen der allgemeinen Formeln (II) und/oder (III)
R-CH=CH-CH₂-E (II)
R¹-CH=CH-CH₂-E¹ (III)
mit den Bedeutungen
E, E¹ unabhängig voneinander -CHO, -COOH, -COOR², -C(O)NR³R⁴, -CN
R, R¹ unabhängig voneinander H, C₁₋₁₂-Alkyl, C₆₋₁₂-Aryl oder C₇₋₁₃-Alkylaryl
R², R³, R⁴ unabhängig voneinander H, C₁₋₁₂-Alkyl, C₇₋₁₃-Aralkyl,
in Gegenwart eines Katalysators,
**dadurch gekennzeichnet, daß** ein homogener Katalysator, der Rutheniumverbindungen oder -komplexe enthält, eingesetzt wird.

2. Verfahren nach Anspruch 1, **dadurch gekennzeichnet, daß** als Katalysator Ruthenium-Alkylidenkomplexe eingesetzt werden.

3. Verfahren nach Anspruch 2, **dadurch gekennzeichnet, daß** die Ruthenium-Alkylidenkomplexe ausgewählt sind aus kationischen Rutheniumkomplexen der allgemeinen Formeln A oder B oder diese enthaltenden Gemischen 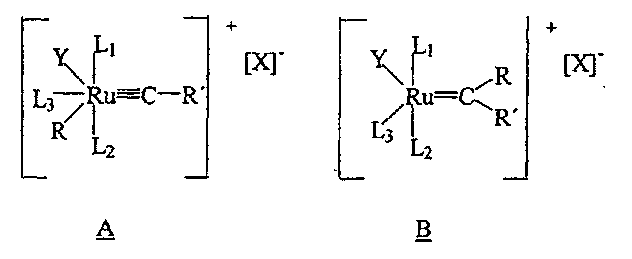 wobei B durch einen weiteren Liganden L₄ stabilisiert sein kann und
X ein nicht oder schwach an das Metallzentrum koordinierendes Anion,
Y einen ein- oder mehrzähnigen anionischen Liganden,
R und R' jeweils unabhängig voneinander Wasserstoff oder einen, gegebenenfalls substituierten, C₁₋₂₀-Alkyl-, C₆₋₂₀-Aryl- oder C₇₋₂₀-Alkylarylrest und
L₁, L₂, L₃ und L₄ unabhängig voneinander neutrale Elektronendonor-Liganden bedeuten,
oder
Rutheniumkomplexen der allgemeinen Formeln C oder D
RuX'Y'(=CH-CH₂R")L¹L² (C)
RuX'Y'(=CHR")L¹L² (D)
in der
X', Y' die gleichen oder unterschiedliche anionische Liganden sind,
R" Wasserstoff oder ein, gegebenenfalls substituierter, C₁₋₂₀-Alkylrest oder C₆₋₂₀-Arylrest ist, und
L¹ und L² unabhängig voneinander neutrale Elektronendonor-Liganden sind.

4. Verfahren nach Anspruch 3, **dadurch gekennzeichnet, daß** die neutralen Elektronendonor-Liganden Phosphane, Arsane, Stibane mit mindestens zwei sterisch anspruchsvollen Resten, Amine, Pyridine, π-koordinierte Olefine oder Lösemittelmoleküle sind.

5. Verfahren nach Anspruch 4, **dadurch gekennzeichnet, daß** die neutralen Elektronendonor-Liganden ausgewählt sind aus Phosphanen der allgemeinen Formel
PR^{a}R^{b}R^{c}
in der R^{a} und R^{b} unabhängig voneinander Phenylreste oder organische sterisch hindernde Reste sind und R^{c} Wasserstoff, ein, gegebenenfalls substituierter, C₁₋₁₂-Alkylrest oder C₆₋₂₀-Arylrest oder wie für R^{a} definiert ist.

6. Verfahren nach einem der Ansprüche 3 bis 5, **dadurch gekennzeichnet, daß** X Halogen und Y das gleiche oder unterschiedliches Halogen oder ein an ein Polymer oder einen Träger gebundenes Carboxyl bedeuten.

7. Verfahren nach einem der Ansprüche 1 bis 6, **dadurch gekennzeichnet, daß** E gleich E' und R gleich R' ist.

8. Verfahren nach einem der Ansprüche 1 bis 7, **dadurch gekennzeichnet, daß** die Umsetzung in Gegenwart von Ethylen durchgeführt wird.

9. Verfahren nach einem der Ansprüche 1 bis 8, **dadurch gekennzeichnet, daß** R-CH=CH-CH₂-E 3-Pentensäuremethylester oder 3-Pentensäure bedeutet.

## Claims

1. A process for preparing C₆ compounds of the formula (I)
E-CH₂-CH=CH-CH₂-E¹ (I)
by self metathesis or cross metathesis of compounds of the formulae (II) and/or (III)
R-CH=CH-CH₂-E (II)
R¹-CH=CH-CH₂-E¹ (III)
where
E, E¹ are independently -CHO, -COOH, -COOR², -C(O)NR³R⁴, -CN,
R, R¹ are independently H, C₁₋₁₂-alkyl, C₆₋₁₂-aryl or C₇₋₁₃-alkylaryl and
R², R³, R⁴ are independently H, C₁₋₁₂-alkyl, C₇₋₁₃-aralkyl,
in the presence of a homogeneous catalyst comprising ruthenium compounds or ruthenium complexes.

2. A process as claimed in claim 1, wherein ruthenium-alkylidene complexes are used as catalyst.

3. A process as claimed in claim 2, wherein the ruthenium-alkylidene complexes are selected from among cationic ruthenium complexes of the formula A or B or mixtures comprising them 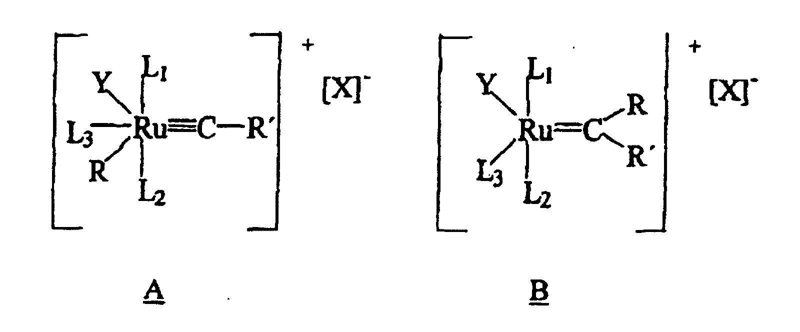 where B can be stabilized by a further ligand L₄ and
X is an anion which does not coordinate or coordinates only weakly to the metal center,
Y is a monodentate or polydentate anionic ligand,
R and R' are each, independently of one another, hydrogen or a substituted or unsubstituted C₁₋₂₀-alkyl, C₆₋₂₀-aryl or C₇₋₂₀-alkylaryl radical and
L₁, L₂, L₃ and L₄ are, independently of one another, uncharged electron donor ligands,
or
ruthenium complexes of the formulae C or D
RuX'Y' (=CH-CH₂R")L¹L² (C)
RuX'Y' (=CHR")L¹L² (D)
where
X', Y' are identical or different anionic ligands,
R" is hydrogen or a substituted or unsubstituted C₁₋₂₀-alkyl radical or C₆₋₂₀-aryl radical, and
L¹ and L² are, independently of one another, uncharged electron donor ligands.

4. A process as claimed in claim 3, wherein the uncharged electron donor ligands are phosphines, arsines, stibines containing at least two bulky groups, amines, pyridines, π-coordinated olefins or solvent molecules.

5. A process as claimed in claim 4, wherein the uncharged electron donor ligands are selected from among phosphines of the formula
PR^{a}R^{b}R^{c}
where R^{a} and R^{b} are, independently of one another, phenyl radicals or sterically hindered organic radicals and R^{c} is hydrogen, a substituted or unsubstituted C₁₋₁₂-alkyl radical or C₆₋₂₀-aryl radical or is as defined for R^{a}.

6. A process as claimed in any of claims 3 to 5, wherein X is halogen and Y is the same halogen or a different halogen or a carboxyl group bound to a polymer or a support.

7. A process as claimed in any of claims 1 to 6, wherein E is identical to E' and R is identical to R'.

8. A process as claimed in any of claims 1 to 7, wherein the reaction is carried out in the presence of ethylene.

9. A process as claimed in any of claims 1 to 8, wherein R-CH=CH-CH₂-E is methyl 3-pentenoate or 3-pentenoic acid.

## Revendications

1. Procédé de préparation de composés en C₆ de formule générale (I)
E-CH₂-CH=CH-CH₂-E¹ (I)
au moyen d'une autométathèse ou d'une métathèse croisée, à partir des composés de formules générales (II) et/ou (III)
R-CH=CH-CH₂-E (II)
R¹-CH=CH-CH₂-E¹ (III)
dans lesquelles
E, E¹ représentent, indépendamment l'un de l'autre, un reste -CHO, -COOH, -COOR², -C(O)NR³R⁴ ou -CN,
R, R¹ représentent, indépendamment l'un de l'autre, un atome H, un groupe alkyle en C₁ à C₁₂, aryle en C₆ à C₁₂, ou alkylaryle en C₇ à C₁₃,
R², R³, R⁴ représentent, indépendamment l'un de l'autre, un atome H, un groupe alkyle en C₁ à C₁₂ ou aralkyle en C₇ à C₁₃,
en présence d'un catalyseur,
**caractérisé en ce que** l'on met en oeuvre un catalyseur homogène comprenant des complexes ou des composés de ruthénium.

2. Procédé selon la revendication 1, **caractérisé en ce que** l'on met en oeuvre, en tant que catalyseur, les complexes alkylidènes de ruthénium.

3. Procédé selon la revendication 2, **caractérisé en ce que** l'on choisit les complexes alkylidènes de ruthénium dans le groupe formé par les complexes de ruthénium cationiques de formules générales A et B et les mélanges contenant ces derniers 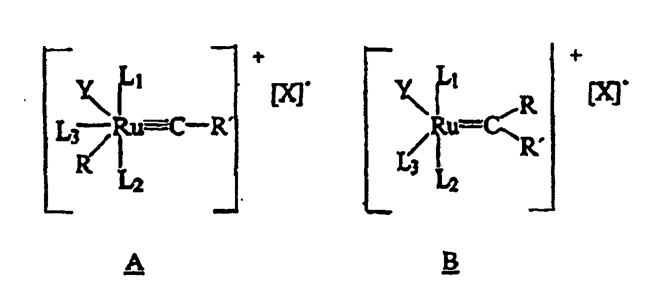 où B est éventuellement stabilisé par un ligand L₄ supplémentaire et
x représente un anion qui n'est pas coordonné au centre métallique ou alors de manière faible,
Y représente un ligand anionique monodenté ou polydenté,
R et R' représentent, à chaque fois indépendamment l'un de l'autre, un atome d'hydrogène ou un reste alkyle en C₁ à C₂₀, aryle en C₆ à C₂₀ ou alkylaryle en C₇ à C₂₀, reste qui est éventuellement substitué, et
L₁, L₂, L₃, et L₄ représentent, indépendamment les uns des autres, un ligand donneur d'électrons neutre.
ou
dans le groupe formé par les complexes de ruthénium de formules générales C et D
RuX'Y' (=CH-CH₂R")L¹L² (C)
RuX'Y' (=CHR")L¹L² (D)
dans lesquelles
X', Y' sont identiques ou différents et représentent un ligand anionique,
R'' représente un atome d'hydrogène ou un reste aryle en C₆ à C₂₀ ou un reste alkyle en C₁ à C₂₀ qui est éventuellement substitué, et
L¹ et L² représentent, indépendamment l'un de l'autre, un ligand donneur d'électrons.

4. Procédé selon la revendication 3, **caractérisé en ce que** les ligands donneurs d'électrons neutres sont choisis parmi les phosphanes, les arsanes, les stilbanes présentant au moins deux restes à encombrement stérique, les amines, les pyridines, les oléfines à coordination π et les molécules de solvant.

5. Procédé selon la revendication 4, **caractérisé en ce que** l'on choisit les ligands donneurs d'électrons neutres parmi les phosphanes de formule générale
PR^{a}R^{b}R^{c}
dans laquelle R^{a} et R^{b} représentent, indépendamment l'un de l'autre, un reste phényle ou un reste organique à encombrement stérique, et R^{c} représente un atome d'hydrogène, un reste aryle en C₆ à C₂₀ ou un reste alkyle en C₁ à C₁₂ qui est éventuellement substitué, ou bien prend la signification de R^{a}.

6. Procédé selon l'une quelconque des revendications 3 à 5, **caractérisé en ce que** X représente un atome d'halogène et Y représente soit un atome d'halogène identique ou différent soit un groupe carboxyle lié à un polymère ou à un support.

7. Procédé selon l'une quelconque des revendications 1 à 6, **caractérisé en ce que** E est identique à E' et R est identique à R'.

8. Procédé selon l'une quelconque des revendications 1 à 7, **caractérisé en ce que** l'on procède à la réaction en présence d'éthylène.

9. Procédé selon l'une quelconque des revendications 1 à 8, **caractérisé en ce que** R-CH=CH-CH₂-E représente l'ester méthylique de l'acide pentène-3-oïque ou l'acide pentène-3-oïque.
